(19) Europäisches Patentamt
European Patent Office
Office européen des brevets

(11) EP 0 529 601 B1

(12) EUROPÄISCHE PATENTSCHRIFT

(45) Veröffentlichungstag und Bekanntmachung des Hinweises auf die Patenterteilung:
**23.07.1997 Patentblatt 1997/30**

(51) Int Cl.$^6$: **C12P 41/00**

(21) Anmeldenummer: **92114528.0**

(22) Anmeldetag: **26.08.1992**

(54) **Verfahren zur Herstellung von optisch aktivem Vinylglycin in freier oder geschützter Form über eine enzymatische, enantioselektive Hydrolyse**

Process for the production of optically active vinylglycine in free or protected form by an enzymatic, enantioselective hydrolysis

Procédé de production de vinylglycine optiquement active en forme libre ou protégée par une hydrolyse enzymatique et enautioselective

(84) Benannte Vertragsstaaten:
**AT BE CH DE DK ES FR GB GR IT LI NL PT SE**

(30) Priorität: **30.08.1991 DE 4128956**

(43) Veröffentlichungstag der Anmeldung:
**03.03.1993 Patentblatt 1993/09**

(73) Patentinhaber: **HOECHST AKTIENGESELLSCHAFT
65926 Frankfurt am Main (DE)**

(72) Erfinder:
- **Fülling, Gerd, Dr.
W-6232 Bad Soden am Taunus (DE)**
- **Kretzschmar, Gerhard, Dr.
W-6236 Eschborn 2 (DE)**

(56) Entgegenhaltungen:
**DE-A- 2 927 534** **US-A- 4 806 680**

- **SYNTHETIC COMMUNICATIONS, Band 19, Nr. 20, 1989, Columbia (US); P. MEFFRE et al., Seiten 3457-3468/**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 529 601 B1

Printed by Jouve, 75001 PARIS (FR)

## Beschreibung

Verfahren zur Herstellung von optisch aktivem Vinylglycin in freier oder geschützter Form über eine enzymatische, enantioselektive Hydrolyse.

Die Erfindung betrifft die Synthese der optisch aktiven Formen von Vinylglycin bzw. seiner Derivate in freier oder geschützter Form.

Vinylglycin ist der einfachste Vertreter der Klasse der 3,4-Dehydroaminosäuren. Diese Verbindungsklasse, und insbesondere Vinylglycin, ist aufgrund ihrer Enzyminhibitoreigenschaften von Interesse [Meffre et al., Synthetic Communications 19, 3457 (1989)].

Darüber hinaus besitzen Vinylglycin-Derivate als optisch aktive Synthesebausteine zur Herstellung von Natur- und Wirkstoffen eine zentrale Bedeutung. Beispielsweise beschreiben Wade et al. [Tetrahedron Lett. 23,4563 (1982)] die Überführung eines geschützten L-Vinylglycins in Acivicin. Diese Verbindung besitzt Antitumorwirkung. Thornberry et al. [J. Am. Chem. Soc. 109, 7543 (1987)] setzen D-Vinylglycin als Synthesebaustein für den E.-coli-B.-Alanin-Racemase-Inhibitor D-Chlorvinylglycin ein. Shaw et al. [J. Org. Chem. 50, 4515 (1985)] überführen ein D-Vinylglycin-Derivat in Mitomycin-Analoga, eine Verbindungsklasse mit Antitumor-und antibakterieller Wirkung. In DE 3817956 A1 wird die Verwendung geschützer L-Vinylglycin-Derivate zur Synthese von herbiziden, fungiziden und antiviralen phosphorhaltigen L-Aminosäuren beschrieben.

Die Synthese von optisch aktiven Vinylglycin-Vorstufen ist an sich bekannt. Dazu geht man i. a. von geeigneten homochiralen Aminosäuren aus [Pelliciari et al., Synthetic Communications 18, 1715, (1988); Rapoport et al. J. Org. Chem. 45, 4817 (1980)]. Die Konfiguration des Zielmoleküls Vinylglycin ist dabei durch die Stereochemie der eingesetzten Aminosäure vorgegeben. Alle von den optisch reinen Aminosäuren ausgehenden Methoden zur Synthese von optisch aktiven Vinylglycin-Derivaten weisen daher den gemeinsamen Nachteil auf, daß zwar die natürlichen L-Aminosäuren gut verfügbar sind, aber die nicht natürlichen D-Aminosäuren als potentielle Vorläufer für D-Vinylglycin ungleich teurer sind. Die einzig technisch interessante Alternative ist die Synthesevariante des Methioninwegs nach Rapoport [Meffre et al. Synthetic Communications, 19, 3457 (1989)]. Im Fall des Methionins ist aber das großtechnisch synthetisch hergestellte Racemat ungleich besser verfügbar als die optisch reinen Antipoden.

Als Alternative zur Herstellung optisch reiner Vinylglycin-Antipoden wäre eine Synthese von racemischen Vinylglycin-Vorstufen mit anschließender Racematspaltung denkbar. Die Synthese von racemischem Vinylglycin in Anlehnung an klassische Aminosäuresynthesen ist aus der Literatur hinreichend bekannt (s. z. B. Meffre et al., Synthetic Communications, 19, 3457, 1989 und dort zitierte Literatur). Daneben ist es für den Fachmann leicht nachvollziehbar, die oben beschriebenen Methoden zur Herstellung von L-Vinylglycin aus L-Aminosäuren auf entsprechende racemische Synthesevarianten ausgehend von D,L-Aminosäuren auszudehnen.

Allerdings ist noch keine effiziente Methode für die Gewinnung der optisch reinen Antipoden des Vinylglycins bzw. einer geeigneten Vorstufe aus dem Racemat über eine chemische oder biokatalytische Racematspaltung bekannt. Friis et al. [Acta Scandinavica B 28, 317 (1974)] publizierten die Gewinnung des D-Enantiomers über einen L-selektiven Abbau des Vinylglycins mit L-Aminosäure-oxidase. Eine Isolierung des L-Enantiomers aufgrund des Abbaus zur entsprechenden Ketocarbonsäure ist nicht möglich. Friis et al. synthetisierten Vinylglycin in 1 - 7 %iger Ausbeute nach einer konventionellen Aminosäure-Synthese und unterwarfen das Racemat einer L-selektiven enzymatischen Desaminierung. Bei Einsatz einer L-Aminosäureoxidase erhielten sie optisch angereichertes D-Vinylglycin mit ca. 25 % optischer Ausbeute; der L-selektive Abbau von rac-Vinylglycin mit Bäckerhefe lieferte immerhin 82 % ee. Allerdings erscheint der letztgenannte Zugang aufgrund des ungünstigen Verhältnisses von 5 g Bäckerhefe pro 200 mg Vinylglycin technisch ungeeignet zu sein.

Weiterhin bekannt sind Racematspaltungen von Aminosäurederivaten durch enantioselektive Esterhydrolyse oder N-Acylspaltungen unter Einsatz hydrolytischer Enzyme.
Eine stereospezifische Esterhydrolyse von N-geschützten Aminosäureestern durch hydrolytisch aktive Enzyme wird beispielsweise in DE 2927534 beschrieben. Der Einsatz von Acylasen zur Racematspaltung von Aminosäurederivaten wird z.B. von Chenault et al.[J. Am. Chem. Soc. 111, 6354, 1987] beschrieben. Bei dieser Reaktion katalysiert das Enzym Acylase selektiv die Deacylierung des L-Enantiomers. Auf einfache Weise kann das L-Enantiomer vom D-Acetylderivat getrennt werden.

Es ist andererseits auch bekannt, daß 3,4-Dehydroaminosäuren eine Sonderstellung einnehmen und die enzymatische Hydrolyse mit Problemen, beispielsweise Racemisierung während der enzymatischen Hydrolyse, behaftet ist [Havli č ek et al., Collect. Czech. Chem. Commun. 55, 2074, 1990, insbesondere S. 2078].

Es ist zu vermuten, daß die 3,4-Dehydroaminosäuren aufgrund ihrer Eigenschaft als Enzyminhibitoren bzw. aufgrund der besonderen chemischen Reaktivität, wie z.B. der leichten Isomerisierung der Doppelbindung, diese Sonderstellung in der enzymatischen Hydrolyse einnehmen.

Die obengenannten Literaturbeispiele zeigen, daß bei Versuchen zur biokatalytischen Racematspaltung von 3,4-Dehydroaminosäuren eine im industriellen Maßstab effektive enzymatische Hydrolyse von Vinylglycin-Derivaten bisher nicht durchführbar ist.

Die Beispiele zeigen außerdem, daß die biokatalytische Racematspaltung der 3,4-Dehydroaminosäuren nicht entsprechend der erwartenden chemischen Reaktion abläuft, sondern jede Umsetzung eine Sonderstellung einnimmt.

Betreffend die Umsetzung der Vinylglycin-Vorstufen ist beim Vorhandensein von zwei Chiralitätszentren der enzymatische Angriff auf beide Zentren zu erwarten und es entstehen lediglich Diastereomerengemische. Die enzymatische Umsetzung einer Verbindung mit zwei Chiralitätszentren zu reinen Enantiomeren ist nicht bekannt.

Es wurde nun überraschend gefunden, daß sich racemisches Vinylglycin bzw. racemische Vinylglycin-Derivate über eine enzymatische Esterhydrolyse mit hohen chemischen und optischen Ausbeuten in die optischen Antipoden spalten lassen.

Die Erfindung betrifft somit ein Verfahren zur enzymatischen Racematspaltung von Vinylglycin bzw. von Vinylglycin-Vorstufen, das dadurch gekennzeichnet ist, daß

a) ein Gemisch der D- und L-Verbindung der Formel I

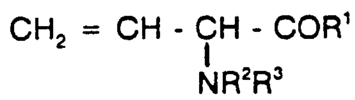 I

in welcher

| | |
|---|---|
| $R^1$ | für unverzweigtes oder verzweigtes $C_1$-$C_{20}$-Alkoxy, das unsubstituiert oder durch ein oder mehrere Halogenreste, wie Fluor, Chlor, Brom oder Jod, oder ein- oder mehrfach durch $C_1$-$C_8$-Alkoxy oder durch Phenyl oder Phenyl, das 1 bis 3 Substituenten aus der Gruppe $C_1$-$C_{12}$-Alkyl, $C_1$-$C_{12}$-Alkoxy, Halogen, Nitro und $CF_3$ trägt, substituiert ist,<br>oder für $C_3$-$C_8$-Cycloalkyl, das durch ein oder mehrere Gruppen aus $C_1$-$C_4$-Alkyl, $C_1$-$C_4$-Alkoxy und Halogen substituiert sein kann,<br>oder für $C_2$-$C_{10}$-Alkenyl oder $C_3$-$C_{10}$-Alkinyl steht;<br>und |
| $R^2$ und $R^3$ | Wasserstoff, Formyl, unverzweigtes oder verzweigtes ($C_1$-$C_{20}$-Alkyl)-carbonyl, das unsubstituiert oder im Alkylteil durch ein oder mehrere Reste aus Hydroxy, Halogen, Phenyl, welches durch bis zu 3 Reste aus der Gruppe $C_1$-$C_{12}$-Alkyl, $C_1$-$C_{12}$-Alkoxy, Halogen, Nitro und $CF_3$ substituiert sein kann oder $R^2$ und $R^3$ bedeuten $C_1$-$C_4$-Alkoxy und $C_1$-$C_4$-Alkylthio,<br>sowie Benzoyl oder Benzoyl, das durch 1 bis 3 Reste aus der Gruppe $C_1$-$C_{12}$-Alkyl, $C_1$-$C_{12}$-Alkoxy, Halogen, Nitro und $CF_3$ substituiert ist oder eine andere für Aminogruppen übliche und dem Fachmann bekannte Schutzgruppe bedeutet |

wobei $R^2$ jeweils gleich oder ungleich $R^3$ ist oder $R^1$ die oben genannten Bedeutungen haben kann und $R^2$ zusammen mit $R^3$ einen Benzol-1,2-dicarbonyl-Rest bildet;
oder

b) die Verbindung der Formel II

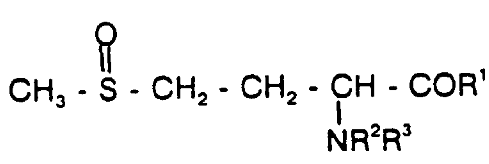 II

in welcher

| | |
|---|---|
| $R^1$, $R^2$ sowie $R^3$ | die unter a) genannten Bedeutungen haben, |

In wäßrigem oder wäßrig-organischem Medium mit einem hydrolytisch aktiven Enzym umgesetzt wird, wobei eine Lipase vorzugsweise jedoch eine Esterase oder eine Protease verwendet wird.

Im folgenden wird die Erfindung erläutert und in ihren Patentansprüche definiert.

Als Ausgangssubstanz wird die Verbindung der Formel I (Vinylglycin und Derivate) oder die Verbindung der Formel II als synthetische Vinylglycin-Vorstufe verwendet, die als ein racemisches Gemisch vorliegt und in welcher die Substituenten $R^1$, $R^2$ und $R^3$ die oben genannten Bedeutungen haben.

Die Verbindung der Formel I wird mit Hilfe des Enzyms in die optische Antipoden gespalten. Dabei wird selektiv das L-Enantiomer der allgemeinen Formel I enzymatisch zur korrespondierenden Aminosäuren der allgemeinen Formel I mit $R^1$ = OH umgesetzt. Das D-Enantiomer der allgemeinen Formel I wird nicht hydrolysiert.

Die Verbindung der Formel II wird zur Racematspaltung entweder in die Verbindung der Formel I übergeführt und wie zuvor beschrieben in die optischen Antipoden gespalten oder die Verbindung der Formel II wird zur Racematspaltung ebenfalls mit dem Enzym umgesetzt, wobei wiederum nur die L-Form der allgemeinen Formel II, zur Verbindung der allgemeinen Formel II mit $R^1$=OH hydrolysiert wird.

Die D-Form wird nicht enzymatisch hydrolysiert und verbleibt als Verbindung der allgemeinen Formel II.

Die Überführung in die Vinylglycin-Derivate der allgemeinen Formel I ist an sich bekannt, wobei die L-Form der allgemeinen Formel II mit $R^1$=OH gegebenenfalls zuvor durch bekannte Methoden der Veresterung in Verbindungen der allgemeinen Formel II übergeführt werden.

Von besonderem Interesse ist ein erfindungsgemäßes Verfahren, das dadurch gekennzeichnet ist, daß man ein Gemisch von D- und L-Vinylglycin bzw. Vinylglycin-Derivaten der Formel I verwendet, in welcher

$R^1$ für unverzweigtes oder verzweigtes $C_1$-$C_8$-Alkoxy, das unsubstituiert oder ein- oder mehrfach durch Fluor oder Chlor oder ein- oder mehrfach durch $C_1$-$C_4$-Alkoxy oder durch Phenyl oder Phenyl, das 1 bis 3 Substituenten aus der Gruppe $C_1$-$C_4$-Alkyl, $C_1$-$C_4$-Alkoxy, Halogen oder Nitro trägt, substituiert ist, steht
und

$R^2$ und $R^3$ für Wasserstoff, Formyl, unverzweigtes oder verzweigtes ($C_1$-$C_{10}$-Alkyl)-carbonyl, das unsubstituiert oder im Alkylteil durch ein bis drei Reste aus Hydroxy, Halogen, Phenyl, welches durch bis zu 3 Reste aus der Gruppe $C_1$-$C_4$-Alkyl, $C_1$-$C_4$-Alkoxy, Halogen, Nitro und $CF_3$ substituiert sein kann, $C_1$-$C_4$-Alkoxy und $C_1$-$C_4$-Alkylthio substituiert ist, für Benzoyl oder Benzoyl, das durch 1 bis 3 Reste aus der Gruppe $C_1$-$C_4$-Alkyl, $C_1$-$C_4$-Alkoxy, Halogen und Nitro substituiert ist, steht oder für eine andere für Aminogruppen übliche und dem Fachmann bekannte Schutzgruppe, insbesondere ausgewählt aus unverzweigtem oder verzweigtem ($C_1$-$C_{20}$-Alkoxy)-carbonyl und $C_1$-$C_{20}$-Alkylsulfonyl, die jeweils unsubstituiert oder im Alkylteil durch ein oder mehrere Reste aus der Gruppe Hydroxy, Halogen, $C_1$-$C_4$-Alkoxy, $C_1$-$C_4$-Alkylthio, Phenyl oder Phenyl, das 1 bis 3 Substituenten aus der Gruppe $C_1$-$C_{12}$-Alkyl, $C_1$-$C_{12}$-Alkoxy, Halogen, Nitro und $CF_3$ trägt, substituiert sind, und Phenylsulfonyl, das durch bis zu drei Resten aus $C_1$-$C_{12}$-Alkyl, $C_1$-$C_{12}$-Alkoxy, Halogen, Nitro und $CF_3$ substituiert sein kann, steht, und ganz insbesondere ausgewählt aus unverzweigtem oder verzweigtem ($C_1$-$C_{10}$-Alkoxy)-carbonyl und $C_1$-$C_{20}$-Alkylsulfonyl, die jeweils unsubstituiert oder im Alkylteil durch ein oder mehrere Reste aus der Gruppe Hydroxy, Halogen, Methoxy, Ethoxy, Phenyl oder einen Phenylrest, der 1 bis 3 Substituenten aus der Gruppe $C_1$-$C_4$-Alkyl, $C_1$-$C_4$-Alkoxy, Halogen und Nitro trägt, substituiert ist, steht,

wobei $R^2$ jeweils gleich oder ungleich $R^3$ ist.

Ganz besonders bevorzugt ist als Ausgangssubstanz ein Gemisch von D- und L-Vinylglycin-Derivaten der genannten Formel I, in welcher

$R^1$ für unverzweigtes oder verzweigtes $C_1$-$C_6$-Alkoxy, vorzugsweise Methoxy, Ethoxy, Isopropyloxy oder Benzyloxy steht
und

$R^2$ und $R^3$ für Wasserstoff, Formyl, unverzweigtes oder verzweigtes ($C_1$-$C_6$-Alkyl)-carbonyl, vorzugsweise Acetyl, das unsubstituiert oder im Alkylteil durch ein bis drei Reste aus Halogen oder Phenyl substituiert sein kann, für Benzoyl oder Benzoyl, das durch 1 bis 3 Reste aus der Gruppe $C_1$-$C_4$-Alkyl, $C_1$-$C_4$-Alkoxy, Halogen und Nitro substituiert ist oder für unverzweigtes oder verzweigtes ($C_1$-$C_4$-Alkoxy)-carbonyl, vorzugsweise Methoxycarbonyl, tert.-Butyloxycarbonyl (BOC) oder Benzyloxycarbonyl (Z), das im Phenylring noch bis zu drei Reste aus der Gruppe $C_1$-$C_4$-Alkyl, $C_1$-$C_4$-Alkoxy und Halogen substituiert sein kann, steht,
wobei

$R^2$ jeweils gleich oder ungleich $R^3$ ist.

$C_1$-$C_6$-Alkyl bedeutet insbesondere Methyl, Ethyl, 1-Propyl oder 2-Propyl, n-, i-, tert- oder 2-Butyl, 3-Methyl-but-2-yl, n-, i-, tert-,2- oder 3-Pentyl, n-Hexyl oder ein stereoisomeres Hexyl, $C_1$-$C_6$-Alkoxy bedeutet insbesondere ($C_1$-$C_6$-Alkyl)-oxy, wobei der Alkylrest dabei die vorstehend genannte Bedeutung besitzt.

Es wurde weiterhin gefunden, daß ebenfalls eine enantioselektive, enzymatische Racematspaltung auf der Vinylglycinvorstufe der allgemeinen Formel II mit hohen Umsatzgeschwindigkeiten sowie hohen chemischen und optischen Ausbeuten erfolgreich durchgeführt werden kann, wobei selektiv das L-Enantiomer hydrolysiert wird.

Die Definition der Substituenten $R^2$ und $R^3$ wie sie für die Verbindung der Formel I vorgenommen wurde, insbesondere auch die Darstellung der bevorzugten Substituenten, ist auch für die Verbindung der Formel II gültig.

Die Bezeichnung D bzw. L bei der Vinylglycin-Vorstufe der allgemeinen Formel II gibt nur die Konfiguration an dem in α-Stellung zur Carbonsäuregruppe befindlichen C-Atom an, das mit der geschützten Aminogruppe verbunden ist. Wegen des zusätzlichen Chiralitätszentrums am Schwefelatom sind die Vinylglycin-Vorstufen (Methioninsulfoxid-Derivate) der allgemeinen Formel II in der Regel keine racemischen Gemische, sondern Gemische von Diastereomeren. Allerdings übte überraschenderweise das chirale Schwefelatom keinen Einfluß auf die enzymatische Racematspaltung aus. Die Enzymhydrolyse bricht exakt bei 50 % Umsatz ab, d. h. nur die L-konfigurierten Aminosäuren der diastereomen Gemische des jeweils eingesetzten Methioninsulfoxid-Derivats der allgemeinen Formel II wurden enzymatisch hydrolysiert.

Die Synthese der D,L-Vinylglycin-Vorstufen erfolgt in Anlehnung an literaturbekannte Verfahren [Meffre et al., s. o.] mit der Ausnahme, daß das dort beschriebene Verfahren ausgehend von L-Methionin auf eine racemische Variante, d. h. ausgehend von D,L-Methionin, ausgedehnt wurde.

Für die erfindungsgemäße enantioselektive enzymatische Hydrolyse der Carbonsäureesterfunktion an den Verbindungen der allgemeinen Formel I und II kommen hydrolytische, insbesondere proteolytische und esteraseaktive Enzyme in Frage, insbesondere Serin- und Sulfhydryl-Proteasen, wie beispielsweise Subtilisin (EC 3.4.4.16), α-Chymotrypsin (EC 3.4.4.5), Papain (EC 3.4.4.10), Ficin, Bromelain oder Thermitase. Bevorzugt sind technische Enzymqualitäten, wie sie beispielsweise unter den Firmennamen Maxatase® (Hersteller Gist-Brocades N.V., Delft/ Niederlande) und Alcalase® (Hersteller NOVO, Kopenhagen/Dänemark) bekannt sind.

Die besagten Enzyme können in freier Form oder nach Immobilierung gemäß dem Fachmann bekannten oder üblichen Verfahren [Mosbach, Methods Enzymol. XLIV, Immobilized Enzymes, Academic Press, New York 1976] eingesetzt werden. Das jeweilige Substrat wird im wäßrigen Milieu gelöst oder suspendiert eingesetzt. Konzentrationen von 0,1 % bis zur gesättigten Lösung sind ebenso möglich wie das Arbeiten in Suspension (über Sättigungsgrenze hinausgehende Einsatzmenge an Substrat). Der Zusatz von wasserlöslichen Lösungsmitteln wie beispielsweise Methanol ist im Einzelfall ebenso praktizierbar wie das Arbeiten im Zweiphasengebiet unter Zusatz wasserunlöslicher organischer Lösungsmittel.

Die Reaktionstemperatur für die enzymatischen Spaltungen liegt in der Regel zwischen 10 und 80 °C, bevorzugt zwischen 20 und 50 °C. Das Verfahren kann als Batchverfahren oder kontinuierlich als Säulenverfahren durchgeführt werden.

Die enzymatische Hydrolyse wird in gepufferter Lösung oder in destilliertem Wasser durchgeführt.

Die Pufferlösung sowie deren Konzentration sind dem Fachmann bekannt. Es kann z.B. Kalium-Phosphat-Puffer, 0,01 - 5 M, verwendet werden.

Die enzymatische Hydrolyse wird vorzugsweise bei einem pH von 5 bis 10, insbesondere pH 6 bis 9 durchgeführt. Dabei werden nur die L-Vinylglycin-Derivate bzw. Vorstufen der allgemeinen Formel I und II als Substrat von dem Enzym akzeptiert und hydrolysiert, nicht aber die D-Enantiomeren.

Der Verlauf der Reaktion kann beispielsweise über HPLC anhand der Abnahme des Substrats oder anhand der Zunahme des Produkts verfolgt werden. Alternativ ist eine Bestimmung der gebildeten Menge an Carbonsäure durch die Menge an erforderlicher zudosierter Base bis zur pH-Konstanz möglich.

Die Trennung und Isolierung der Reaktionsprodukte, d. h. der D-Vinylglycin-Vorstufen der allgemeinen Formel I und II sowie der L-Vinylglycin-Vorstufen der allgemeinen Formel I und II mit $R^1$=OH gelingt nach im Prinzip bekannter oder üblicher Weise, wie beispielsweise durch Kristallisation, Destillation, Säulenchromatographie (Adsorption, Ionenaustausch) und insbesondere durch Extraktion. Dazu wird die Reaktionslösung auf einen pH- Wert zwischen 6 und 12 eingestellt, so daß das Hydrolyseprodukt als Carboxylatsalz ($R^1$ = $O^-M^+$) vorliegt und der nicht umgesetzte D-Vinylglycin-Ester der allgemeinen Formel I oder II mit einem geeigneten organischen Lösungsmittel extrahiert werden kann, wobei das L-Derivat als Carboxylatsalz in der wäßrigen Phase verbleibt. Dabei ist der pH-Wert der wäßrigen Phase so zu wählen, daß einerseits die vollständige Bildung des Carboxylatsalzes gewährleistet ist und andererseits aber noch keine chemische Hydrolyse der Carboxylester Funktion der Verbindungen der allgemeinen Formel I oder II auftritt. Nach Abtrennung des D-Derivats wird die wäßrige Lösung mit Mineralsäure, beispielsweise Salzsäure oder Schwefelsäure, auf pH < 5 gestellt, und das L-Derivat der allgemeinen Formel I oder II mit $R^1$ = OH als freie Säure mit einem geeigneten Lösungsmittel extrahiert.

Die getrennten isolierten D- und L-Vinylglycin-Derivate und Vorstufen lassen sich, ggf. nach weiterer chemischen Reinigung durch Säulenchromatographie, Kristallisation oder Destillation auf bekanntem Weg [s. eingangs zitierte

Literatur] in das freie D- bzw. L-Vinylglycin überführen, wobei die L-Vinylglycin-Vorstufen der allgemeinen Formel II mit $R^1$ = OH ggf. nach einem allgemein bekannten Verfahren der Estersynthese, beispielsweise Mineralsäure/Alkohol, in die Verbindung der allgemeinen Formel II überführt werden, bevor sie nach dem an sich literaturbekannten Verfahren in die optisch aktiven Verbindungen der allgemeinen Formel I überführt werden.

Die optische Reinheit der D- bzw. der L-Verbindung läßt sich beispielsweise nach Entfernung der Schutzgruppen zur freien Aminosäure nach einem für Aminosäuren bekannten HPLC- Analysenverfahren feststellen. Dazu wird die aus der Reaktion hervorgegangene und entschützte Aminosäure mit o-Phthalaldehyd und einem optisch-aktiven Thiol, beispielsweise BOC-Cystein oder Acetylcystein nach im Prinzip bekannter Weise [D. Asward, Analytical Biochemistry 137, 405 (1984)] derivatisiert, wonach die D- und die L-Enantiomeren unterschiedliche Retentionszeiten aufweisen. Alternativ wurde die jeweilige Synthesesequenz zu den Verbindungen der allgemeinen Formel I und II ausgehend von optisch reinen L-Aminosäuren durchgeführt und die aus der Racematspaltung hervorgegangenen und isolierten Vinylglycin-Vorstufen anhand des optischen Drehvermögens auf die optische Reinheit überprüft.

Das reine D- bzw. L-Enantiomer kann als Enzyminhibitor verwendet werden. Die D- bzw. L-Enantiomere haben außerdem eine antibakterielle bzw. cytostatische Wirkung.

Die Erfindung wird durch die nachfolgend aufgeführten Beispiele erläutert.

Beispiel 1

4,9 g (19,7 mmol) D,L-Z-Vinylglycinmethylester werden in 20 ml 0,1 M Kaliumphosphat-Puffer, pH 7 suspendiert und nach Zugabe von 800 mg Alcalase® 0,6 (NOVO) für 3 h bei 30 °C gerührt. Der pH-Wert wird während der Reaktion durch kontinuierliches Zudosieren von ca. 1 N NaOH auf pH 7 gehalten. Nach einem Verbrauch von 8,13 ml wird die Reaktion beendet und die Reaktionslösung auf pH 8 gestellt. Die Reaktionslösung wird mehrfach mit $CH_2Cl_2$ extrahiert. Die vereinigten organischen Phasen werden 1x mit wäßriger $NaHCO_3$-Lösung gewaschen, über $Na_2SO_4$ getrocknet und am Rotationsverdampfer i. Vak. zur Trockne eingeengt. Man erhält den D-Z-Vinylglycinester als Öl. Die wäßrige Phase aus der ersten $CH_2Cl_2$-Extraktion wird mit HCl auf pH 2 gestellt und mit $CH_2Cl_2$ extrahiert. Die vereinigten organischen Phasen aus dem zweiten Extraktionsschritt werden über $Na_2SO_4$ getrocknet und i. Vak. zur Trockne eingeengt. Man erhält reines L-Z-Vinylglycin als weißen Feststoff.

| | |
|---|---|
| D-Z-Vinylglycinmethylester: | 1,79 g (37 %) |
| ee (Bestimmung entsprechend Beispiel 2) | ≥ 90 % |
| $^1$H-NMR (100 MHz, $CDCl_3$): | δ = 3,78 (s, 3H, COOMe); 4,92 (m, 1H, CH-N), 5,10 (s, 2H, $CH_2$Ph); 5,30 (m, 2H, $CH_2$=CH); 5,48 (m, br, 1H, NH); 5,90 (m, 1H, $CH_2$=CH), 7,37 (s, 5H, Ph) |
| L-Z-Vinylglycin: | 1,72 g (37 %) |
| ee (Bestimmung s. Beispiel 2): | ≥ 96 % |
| $^1$H-NMR (100 MHz, CDCl3): | δ = 4,95 (m, 1H, CH); 5,13 (s, 2H, $CH_2$Ph); 5,37 (m, 3H, $CH_2$=CH u. NH); 5,91 (m, 1H, $CH_2$=CH); 7,35 (s, 5H, Ph); |

Beispiel 2

25 mg L-Z-Vinylglycin aus Beispiel 1 werden in 2,5 ml 6 N HCl gelöst und für 10-30 min unter Rückfluß zum Sieden erhitzt. Von der Reaktionsmischung wird eine 100-µl-Probe entnommen, mit $NaHPO_4$/NaOH-Lösung, pH 10, auf 5 ml aufgefüllt und mit 67 µl einer Lösung von 500 mg Boc-Cystein in 5 ml Ethanol sowie 33 µl einer Lösung von 333 mg o-Phtaldialdehyd in 5 ml EtOH versetzt. Nach 10 min analysiert man per HPLC (Säule: LiChrosorb® RP-8 [Merck Darmstadt Cat. 15540, LiChroCART® 250-4]; Laufmittel: 0,1 N Kaliumphosphatpuffer (pH 7)/Methanol = 60:40; Fluß 1 ml/min; Detektion: 334 nm; Retentionszeiten: 8,6 min für L-Vinylglycin, 10,7 min für D-Vinylglycin).
In der Probe wird nur ein Peak bei 8,6 min beobachtet.
ee ≥ 96 %

Beispiel 3

2,74 g (11 mmol) Z-D,L-Vinylglycinmethylester werden in 48 ml 0,1 M Kaliumphosphat-Puffer, pH 7 suspendiert und nach Zugabe von 64 mg Alcalase® 0.6 (NOVO) für 1,5 h bei 30 °C gerührt. Der pH-Wert wird während der Reaktion durch kontinuierliches Zudosieren von ca. 0,1 N NaOH auf pH 7 gehalten. Nach einem Verbrauch von 50,2 ml wird die Reaktion beendet und die Reaktionslösung, wie in Beispiel 1 beschrieben, aufgearbeitet.

| | |
|---|---|
| D-Z-Vinylglycinmethylester: | 1,54 g (56 %) |
| ee (Bestimmung entsprechend Beispiel 2): | 75 % |

| | |
|---|---|
| L-Z-Vinylglycin: | 0,95 g (36 %) |
| ee (Bestimmung analog Beispiel 2): | ≥ 96 % |

$^1$H-NMR (100 MHz, $CDCl_3$) jeweils entsprechend Beispiel 1

Beispiel 4

5 g (15,9 mmol) Z-D,L-Methioninsulfoxidmethylester werden in 70 ml 0,1 M Kaliumphosphat-Puffer, pH 7 gelöst und nach Zugabe von 92 mg Alcalase® 0.6 (NOVO) für 2,5 h bei 30 °C gerührt. Der pH-Wert wird während der Reaktion durch kontinuierliches Zudosieren von ca. 0,09 N NaOH auf pH 7 gehalten. Nach einem Verbrauch von 83,3 ml wird die Reaktion beendet und die Reaktionslösung wird entsprechend Beispiel 1 aufgearbeitet.

| | |
|---|---|
| Z-D-Methioninsulfoxid-methylester: | 1,9 g (38 %) |
| $[\alpha]_D^{20}$: | -13,2 ($CHCl_3$) |
| $^1$H-NMR (100 MHz, $CDCl_3$): | δ = 2,27 (m, 2H, $CH_2$-$CH_2$); 2,51, (s, 3H, MeSO); 2,76 (m, 2H, $CH_2$-$CH_2$); 3,73 (s, 3H, COOMe); 4,47 (m, 1H, CH-N); 5,08 (s, 2H, $CH_2$Ph); 5,88 (m, 1H, NH); 7,35 (s, 5H, Ph) |
| Z-L-Methioninsulfoxid: | 2,2 g (46 %) |
| $[\alpha]_D^{20}$ : | +25 ($CHCl_3$) |
| $^1$H-NMR (100 MHz, $CDCl_3$): | δ = 2,32 ((m, 2H, $CH_2$-$CH_2$); 2,64 (s, 3H, MeSO); 2,85 (m, 2H, $CH_2$-$CH_2$); 4,48 (m, 1H, CH-N); 5,08 (s, 2H, $CH_2$Ph); 5,95 (m, 1H, NH); 7,31 (s, 5H, Ph); 8,38 (s, br, COOH) |

Beispiel 5

20 g (64 mmol) Z-D,L-Methioninsulfoxidmethylester werden in 50 ml 0,1 M Kaliumphosphat-Puffer, pH 7 gelöst und nach Zugabe von 1 g Alcalase® 0.6 (NOVO) für 6 h bei 30 °C gerührt. Der pH-Wert wird während der Reaktion durch kontinuierliches Zudosieren von ca. 1 N NaOH auf pH 7 gehalten. Nach einem Verbrauch von 31,2 ml wird die Reaktion beendet und die Reaktionslösung, wie in Beispiel 4 beschrieben, aufgearbeitet.

| | |
|---|---|
| Z-D-Methioninsulfoxid-methylester: | 9,8 g (49 %) |
| $[\alpha]_D^{20}$ : | -11 ($CHCl_3$) |
| Z-L-Methioninsulfoxid: | 9,5 g (50 %) |
| $[\alpha]_D^{20}$ : | +24 ($CHCl_3$) |

$^1$H-NMR (100 MHz, $CDCl_3$) jeweils analog Beispiel 4

Beispiel 6

1 g (3,2 mmol) Z-D,L-Methioninsulfoxidmethylester wird in 40 ml 0,1 M Kaliumphosphat-Puffer, pH 7 gelöst und nach Zugabe von 20 µl Maxatase® (Gist-Brocades) bei 30 °C gerührt. Der pH-Wert wird während der Reaktion durch kontinuierliches Zudosieren von ca. 0,1 N NaOH auf pH 7 gehalten. Nach einem Verbrauch von 16,9 ml 0,1 N NaOH wird die Reaktion beendet und die Reaktionslösung, wie in Beispiel 4 beschrieben, aufgearbeitet.

| | |
|---|---|
| Z-D-Methioninsulfoxid-methylester: | 0,51 g (51 %) |
| $[\alpha]_D^{20}$ : | -14 ($CHCl_3$) |
| Z-L-Methioninsulfoxid: | 0,25 g (26 %) |
| $[\alpha]_D^{20}$ : | +25 ($CHCl_3$) |

$^1$H-NMR (100 MHz, $CDCl_3$) jeweils analog Beispiel 4

Beispiel 7

340 mg (1,14 mmol) D,L-Phenacetylmethioninsulfoxidmethylester werden in 10 ml 0,1 N Kaliumphosphatpuffer, pH7 aufgenommen und nach Zugabe von 50 mg Alcalase® für 1 h bei 30 °C gerührt. Der Umsatz wird durch kontinuierliches Zudosieren von 0,05 N NaOH verfolgt. Die Reaktion stoppt bei einem Verbrauch von 9,4 ml 0,05 N NaOH, d.h. bei einem Umsatz von 50 % des Racemats bzw. 100 % Umsatz des L-Enantiomeren.

## Patentansprüche

1. Verfahren zur enzymatischen Racematspaltung von Vinylglycin bzw. von Vinylglycin-Vorstufen, dadurch gekennzeichnet, daß ein Gemisch der D- und L-Verbindung

   a) der Formel I

   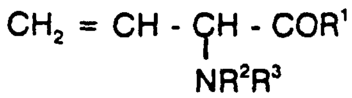

   I

   in welcher

   $R^1$ für unverzweigtes oder verzweigtes $C_1$-$C_{20}$-Alkoxy, das unsubstituiert oder durch ein oder mehrere Halogenreste, wie Fluor, Chlor, Brom oder Jod, oder ein- oder mehrfach durch $C_1$-$C_8$-Alkoxy oder durch Phenyl oder Phenyl, das 1 bis 3 Substituenten aus der Gruppe $C_1$-$C_{12}$-Alkyl, $C_1$-$C_{12}$-Alkoxy, Halogen, Nitro und $CF_3$ trägt, substituiert ist,
   oder für $C_3$-$C_8$-Cycloalkyl, das durch ein oder mehrere Gruppen aus $C_1$-$C_4$-Alkyl, $C_1$-$C_4$-Alkoxy und Halogen substituiert sein kann,
   oder für $C_2$-$C_{10}$-Alkenyl oder $C_3$-$C_{10}$-Alkinyl steht;

   und

   $R^2$ und $R^3$ Wasserstoff, Formyl, unverzweigtes oder verzweigtes ($C_1$-$C_{20}$-Alkyl)-carbonyl, das unsubstituiert oder im Alkylteil durch ein oder mehrere Reste aus Hydroxy, Halogen, Phenyl, welches durch bis zu 3 Reste aus der Gruppe $C_1$-$C_{12}$-Alkyl, $C_1$-$C_{12}$-Alkoxy, Halogen, Nitro und $CF_3$ substituiert sein kann oder $R^2$ und $R^3$ bedeuten $C_1$-$C_4$-Alkoxy und $C_1$-$C_4$-Alkylthio,
   sowie Benzoyl oder Benzoyl, das durch 1 bis 3 Reste aus der Gruppe $C_1$-$C_{12}$-Alkyl, $C_1$-$C_{12}$-Alkoxy, Halogen, Nitro und $CF_3$ substituiert ist oder eine andere für Aminogruppen übliche und dem Fachmann bekannte Schutzgruppe bedeutet

   wobei $R^2$ jeweils gleich oder ungleich $R^3$ ist
   oder $R^1$ die oben genannten Bedeutungen haben kann und $R^2$ zusammen mit $R^3$ einen Benzol-1,2-dicarbonyl-Rest bildet.
   oder

   b) die Verbindung der Formel II

   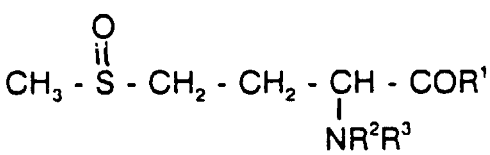

   II

   in welcher

   $R^1$, $R^2$ sowie $R^3$ die unter a) genannten Bedeutungen haben,

   in wäßrigem oder wäßrig-organischem Medium mit einem hydrolytisch aktiven Enzym umgesetzt wird.

2. Verfahren nach Anspruch 1, dadurch gekennzeichnet, daß das Enzym eine Lipase, vorzugsweise eine Esterase oder Protease ist.

3. Verfahren nach Anspruch 1, dadurch gekennzeichnet, daß

$R^1$ für unverzweigtes oder verzweigtes $C_1$-$C_8$-Alkoxy, das unsubstituiert oder ein- oder mehrfach durch Fluor oder Chlor oder ein- oder mehrfach durch $C_1$-$C_4$-Alkoxy oder durch Phenyl oder Phenyl, das 1 bis 3 Substituenten aus der Gruppe $C_1$-$C_4$-Alkyl, $C_1$-$C_4$-Alkoxy, Halogen oder Nitro trägt, substituiert ist, steht

und

$R^2$ und $R^3$ für Wasserstoff, Formyl, unverzweigtes oder verzweigtes ($C_1$-$C_{10}$-Alkyl)-carbonyl, das unsubstituiert oder im Alkylteil durch ein bis drei Reste aus Hydroxy, Halogen, Phenyl, welches durch bis zu 3 Reste aus der Gruppe $C_1$-$C_4$-Alkyl, $C_1$-$C_4$-Alkoxy, Halogen, Nitro und $CF_3$ substituiert sein kann, $C_1$-$C_4$-Alkoxy und $C_1$-$C_4$-Alkylthio substituiert ist, für Benzoyl oder Benzoyl, das durch 1 bis 3 Reste aus der Gruppe $C_1$-$C_4$-Alkyl, $C_1$-$C_4$-Alkoxy, Halogen und Nitro substituiert ist, steht oder für eine andere für Aminogruppen übliche und dem Fachmann bekannte Schutzgruppe, insbesondere ausgewählt aus unverzweigtem oder verzweigtem ($C_1$-$C_{20}$-Alkoxy)-carbonyl und $C_1$-$C_{20}$-Alkylsulfonyl, die jeweils unsubstituiert oder im Alkylteil durch ein oder mehrere Reste aus der Gruppe Hydroxy, Halogen, $C_1$-$C_4$-Alkoxy, $C_1$-$C_4$-Alkylthio, Phenyl oder Phenyl, das 1 bis 3 Substituenten aus der Gruppe $C_1$-$C_{12}$-Alkyl, $C_1$-$C_{12}$-Alkoxy, Halogen, Nitro und $CF_3$ trägt, substituiert sind, und Phenylsulfonyl, das durch bis zu drei Resten aus $C_1$-$C_{12}$-Alkyl, $C_1$-$C_{12}$-Alkoxy, Halogen, Nitro und $CF_3$ substituiert sein kann, steht, und ganz insbesondere ausgewählt aus unverzweigtem oder verzweigtem ($C_1$-$C_{10}$-Alkoxy)-carbonyl und $C_1$-$C_{20}$-Alkylsulfonyl, die jeweils unsubstituiert oder im Alkylteil durch ein oder mehrere Reste aus der Gruppe Hydroxy, Halogen, Methoxy, Ethoxy, Phenyl oder einen Phenylrest, der 1 bis 3 Substituenten aus der Gruppe $C_1$-$C_4$-Alkyl, $C_1$-$C_4$-Alkoxy, Halogen und Nitro trägt, substituiert ist, steht,

wobei

$R^2$ jeweils gleich oder ungleich $R^3$ ist.

**4.** Verfahren nach einem oder mehreren der oben genannten Ansprüche, dadurch gekennzeichnet, daß

$R^1$ für unverzweigtes oder verzweigtes $C_1$-$C_6$-Alkoxy, vorzugsweise Methoxy, Ethoxy, Isopropyloxy oder Benzyloxy steht

und

$R^2$ und $R^3$ für Wasserstoff, Formyl, unverzweigtes oder verzweigtes ($C_1$-$C_6$-Alkyl)-carbonyl, vorzugsweise Acetyl, das unsubstituiert oder im Alkylteil durch ein bis drei Reste aus Halogen oder Phenyl substituiert sein kann, für Benzoyl oder Benzoyl, das durch 1 bis 3 Reste aus der Gruppe $C_1$-$C_4$-Alkyl, $C_1$-$C_4$-Alkoxy, Halogen und Nitro substituiert ist oder für unverzweigtes oder verzweigtes ($C_1$-$C_4$-Alkoxy)-carbonyl, vorzugsweise Methoxycarbonyl, tert.-Butyloxycarbonyl (BOC) oder Benzyloxycarbonyl (Z), das im Phenylring noch bis zu drei Reste aus der Gruppe $C_1$-$C_4$-Alkyl, $C_1$-$C_4$-Alkoxy und Halogen substituiert sein kann, steht,

wobei

$R^2$ jeweils gleich oder ungleich $R^3$ ist.

**5.** Verfahren nach einem oder mehreren der oben genannten Ansprüche, dadurch gekennzeichnet, daß $C_1$-$C_6$-Alkyl bedeutet insbesondere Methyl, Ethyl, 1-Propyl oder 2-Propyl, n-, i-, tert- oder 2-Butyl, 3-Methyl-but-2-yl, n-, i-, tert-, 2- oder 3-Pentyl, n-Hexyl oder ein stereoisomeres Hexyl, $C_1$-$C_6$-Alkoxy bedeutet insbesondere ($C_1$-$C_6$-Alkyl)-oxy, wobei der Alkylrest dabei die vorstehend genannte Bedeutung besitzt.

**6.** Verfahren nach einem oder mehreren der Ansprüche 1-5, dadurch gekennzeichnet, daß die enzymatische Spaltung bei einer Temperatur von 10 bis 80°C, vorzugsweise von 20 bis 50°C durchgeführt wird.

**7.** Verfahren nach einem oder mehreren der Ansprüche 1 bis 6, dadurch gekennzeichnet, daß die Enzyme in freier oder immobilisierter Form eingesetzt werden.

**8.** Verfahren nach einem oder mehreren der Ansprüche 1 bis 7, dadurch gekennzeichnet, daß über die enzymatische

Hydrolyse des D-Vinylglycin-Derivats der allgemeinen Formel I und die L-Form des Vinylglycin-Derivats der allgemeinen Formel I mit $R^1$ = OH erhalten werden.

**9.** Verfahren nach einem oder mehreren der Ansprüche 1 bis 8, dadurch gekennzeichnet, daß über die enzymatische Hydrolyse die D-Vinylglycin Vorstufe der allgemeinen Formel II und die L-Form des Vinylglycin-Derivats der allgemeinen Formel II mit $R^1$ = OH erhalten werden.

## Revendications

**1.** Procédé pour la résolution enzymatique d'un mélange racémique de vinylglycine ou de précurseurs de la vinylglycine, caractérisé en ce que l'on fait réagir un mélange de composés de configuration D et L

a) de formule I

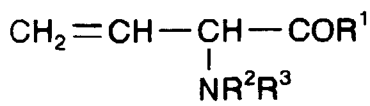

(I)

dans laquelle

$R^1$ représente un groupe alcoxy en $C_1$-$C_{20}$ non ramifié ou ramifié, non substitué ou substitué par un ou plusieurs atomes d'halogène tels que fluor, chlore, brome ou iode, ou par un ou plusieurs groupes alcoxy en $C_1$-$C_8$ ou par un groupe phényle ou un groupe phényle substitué par 1 à 3 substituants choisis parmi les groupes alkyle en $C_1$ -$C_{12}$, alcoxy en $C_1$ -$C_{12}$, halogène, nitro et $CF_3$,
ou un groupe cycloalkyle en $C_3$-$C_8$ qui peut être substitué par un ou plusieurs groupes choisis parmi les groupes alkyle en $C_1$-$C_4$, alcoxy en $C_1$-$C_4$ et halogène,
ou un groupe alcényle en $C_2$-$C_{10}$ ou alcynyle en $C_3$-$C_{10}$ ;

et

$R^2$ et $R^3$ représentent un atome d'hydrogène, un groupe formyle, un groupe (alkyl en $C_1$-$C_{20}$)-carbonyle non ramifié ou ramifié, qui peut être non substitué ou substitué dans la partie alkyle par un ou plusieurs restes choisis parmi les groupes hydroxy, halogène, phényle, celui-ci pouvant porter jusqu'à 3 substituants choisis parmi les groupes alkyle en $C_1$-$C_{12}$, alcoxy en $C_1$-$C_{12}$, halogène, nitro et $CF_3$,
ou $R^2$ et $R^3$ signifient un groupe alcoxy en $C_1$-$C_4$ et un groupe alkylthio en $C_1$-$C_4$,
ainsi qu'un groupe benzoyle ou un groupe benzoyle substitué par 1 à 3 restes choisis parmi les groupes alkyle en $C_1$-$C_{12}$, alcoxy en $C_1$-$C_{12}$, halogène, nitro et $CF_3$ ou un autre groupe protecteur usuel des groupes amino connu du spécialiste,

$R^2$ étant chaque fois identique ou différent de $R^3$
ou bien $R^1$ a les significations données ci-dessus et $R^2$ et $R^3$ forment ensemble un reste benzène-1,2-dicarbonyle

ou bien

b) de formule II

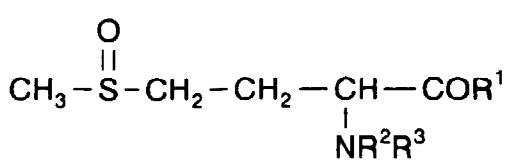

(II)

dans laquelle

$R^1$, $R^2$ ainsi que $R^3$ ont les significations indiquées en a), en milieu aqueux ou aqueux-organique, avec une enzyme à activité hydrolytique.

**2.** Procédé selon la revendication 1, caractérisé en ce que l'enzyme est une lipase, de préférence une estérase ou une protéase.

**3.** Procédé selon la revendication 1, caractérisé en ce que

$R^1$ représente un groupe alcoxy en $C_1$-$C_8$ non ramifié ou ramifié, non substitué ou substitué une ou plusieurs fois par un atome de fluor ou de chlore ou substitué une ou plusieurs fois par un groupe alcoxy en $C_1$-$C_4$ ou par un groupe phényle ou un groupe phényle substitué par 1 à 3 substituants choisis parmi les groupes alkyle en $C_1$-$C_4$, alcoxy en $C_1$-$C_4$, halogène ou nitro

et

$R^2$ et $R^3$ représentent un atome d'hydrogène, un groupe formyle, un groupe (alkyl en $C_1$-$C_{10}$)carbonyle non ramifié ou ramifié, qui peut être non substitué ou substitué dans la partie alkyle par un à trois restes choisis parmi les groupes hydroxy, halogène, phényle, celui-ci pouvant porter jusqu'à 3 substituants choisis parmi les groupes alkyle en $C_1$-$C_4$, alcoxy en $C_1$-$C_4$, halogène, nitro et $CF_3$, un groupe alcoxy en $C_1$-$C_4$ et alkylthio en $C_1$-$C_4$, ainsi qu'un groupe benzoyle ou un groupe benzoyle substitué par 1 à 3 restes choisis parmi les groupes alkyle en $C_1$-$C_4$, alcoxy en $C_1$-$C_4$, halogène et nitro, ou un autre groupe protecteur usuel des groupes amino connu du spécialiste, choisi en particulier parmi les groupes (alcoxy en $C_1$-$C_{20}$)-carbonyle et alkylsulfonyle en $C_1$-$C_{20}$ non ramifié ou ramifié qui peuvent être chacun non substitué ou substitué dans la partie alkyle par un ou plusieurs restes choisis parmi les groupes hydroxy, halogène, alcoxy en $C_1$-$C_4$, alkylthio en $C_1$-$C_4$, phényle ou phényle substitué par 1 à 3 substituants choisis parmi les groupes alkyle en $C_1$-$C_{12}$, alcoxy en $C_1$-$C_{12}$, halogène, nitro et $CF_3$, et le groupe phénylsulfonyle qui peut porter jusqu'à 3 restes choisis parmi les groupes alkyle en $C_1$-$C_{12}$, alcoxy en $C_1$-$C_{12}$, halogène, nitro et $CF_3$, et tout particulièrement choisi parmi des groupes (alcoxy en $C_1$-$C_{10}$)-carbonyle et alkylsulfonyle en $C_1$-$C_{20}$ non ramifié ou ramifié, qui peuvent être chacun non substitué ou substitué dans la partie alkyle par un ou plusieurs restes choisis parmi les groupes hydroxy, halogène, méthoxy, éthoxy, phényle ou phényle substitué par 1 à 3 substituants choisis parmi les groupes alkyle en $C_1$-$C_4$, alcoxy en $C_1$-$C_4$, halogène et nitro,

$R^2$ étant chaque fois identique ou différent de $R^3$.

**4.** Procédé selon une ou plusieurs des revendications indiquées ci-dessus, caractérisé en ce que

$R^1$ représente un groupe alcoxy en $C_1$-$C_6$ non ramifié ou ramifié, de préférence le groupe méthoxy, éthoxy, isopropyloxy ou benzyloxy

et

$R^2$ et $R^3$ représentent un atome d'hydrogène, un groupe formyle, un groupe (alkyl en $C_1$-$C_6$)-carbonyle non ramifié ou ramifié, de préférence un groupe acétyle, qui peut être non substitué ou substitué dans la partie alkyle par un à trois restes choisis parmi des atomes d'halogènes ou un groupe phényle, un groupe benzoyle ou un groupe benzoyle substitué par 1 à 3 restes choisis parmi les groupes alkyle en $C_1$-$C_4$, alcoxy en $C_1$-$C_4$, halogène et nitro, ou un groupe (alcoxy en $C_1$-$C_4$)-carbonyle non ramifié ou ramifié, de préférence les groupes méthoxycarbonyle, tert-butyloxycarbonyle (BOC) ou benzyloxycarbonyle (Z), dont le cycle phényle peut porter jusqu'à trois restes choisis parmi les groupes alkyle en $C_1$-$C_4$, alcoxy en $C_1$-$C_4$ et halogène,

$R^2$ étant chaque fois identique ou différent de $R^3$.

**5.** Procédé selon une ou plusieurs des revendications indiquées ci-dessus, caractérisé en ce que le groupe alkyle en $C_1$-$C_6$ signifie en particulier un groupe méthyle, éthyle, 1-propyle ou 2-propyle, *n-, iso-, tert-* ou 2-butyle, 3-méthyl-but-2-yl, *n-, iso-, tert-,* 2- ou 3-pentyle, *n*-hexyle ou un groupe hexyle stéréoisomère, le groupe alcoxy en $C_1$-$C_6$ signifie en particulier un groupe (alkyl en $C_1$-$C_6$)-oxy, le reste alkyle ayant la signification donnée précédemment.

**6.** Procédé selon une ou plusieurs des revendications 1 à 5, caractérisé en ce que le clivage enzymatique a lieu à une température de 10 à 80 °C, de préférence de 20 °C à 50 °C.

**7.** Procédé selon une ou plusieurs des revendications 1 à 6, caractérisé en ce que l'enzyme est utilisée sous forme libre ou immobilisée.

**8.** Procédé selon une ou plusieurs des revendications 1 à 7, caractérisé en ce que l'on obtient, par hydrolyse enzymatique, le dérivé de la D-vinylglycine de formule générale I et la forme L du dérivé de la vinylglycine de formule I avec $R^1$ = OH.

**9.** Procédé selon une ou plusieurs des revendications 1 à 8, caractérisé en ce que l'on obtient, par hydrolyse enzymatique, le précurseur de la D-vinylglycine de formule générale II et la forme L du dérivé de la vinylglycine de formule générale II avec $R^1$ = OH.

## Claims

**1.** A process for the enzymatic resolution of vinylglycine or of vinylglycine precursors, which comprises reacting a mixture of the D- and L-compound

a) of the formula I

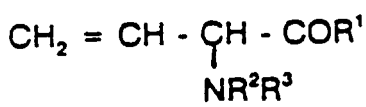

I

in which

$R^1$ is unbranched or branched $C_1$-$C_{20}$-alkoxy, which is unsubstituted or substituted by one or more halogen radicals, such as fluorine, chlorine, bromine or iodine, or mono- or polysubstituted by $C_1$-$C_8$-alkoxy or by phenyl or phenyl which carries 1 to 3 substituents from the group comprising $C_1$-$C_{12}$-alkyl, $C_1$-$C_{12}$-alkoxy, halogen, nitro and $CF_3$,
or is $C_3$-$C_8$-cycloalkyl which can be substituted by one or more $C_1$-$C_4$-alkyl, $C_1$-$C_4$-alkoxy and halogen groups,
or is $C_2$-$C_{10}$-alkenyl or $C_3$-$C_{10}$-alkynyl;

and

$R^2$ and $R^3$ are hydrogen, formyl, unbranched or branched ($C_1$-$C_{20}$-alkyl)carbonyl, which can be unsubstituted or substituted in the alkyl moiety
by one or more hydroxyl, halogen and phenyl radicals, which can be substituted by up to 3 radicals from the group comprising $C_1$-$C_{12}$-alkyl, $C_1$-$C_{12}$-alkoxy, halogen, nitro and $CF_3$ or $R^2$ and $R^3$ are $C_1$-$C_4$-alkoxy and $C_1$-$C_4$-alkylthio,
and also benzoyl or benzoyl which is substituted by 1 to 3 radicals from the group comprising $C_1$-$C_{12}$-alkyl, $C_1$-$C_{12}$-alkoxy, halogen, nitro and $CF_3$ or is another protective group customary for amino groups and known to the person skilled in the art,

where $R^2$ in each case is equal or unequal to $R^3$ or $R^1$ can have the abovementioned meanings and $R^2$, together with $R^3$, forms a benzene-1,2-dicarbonyl radical;
or

b) reacting the compound of the formula II

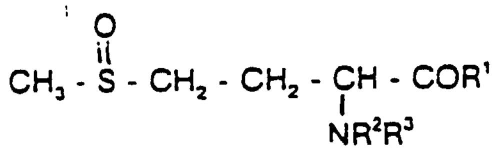

II

in which
$R^1$, $R^2$ and $R^3$ have the meanings mentioned in a),
in aqueous or aqueous-organic medium with a hydrolytically active enzyme.

**2.** The process as claimed in claim 1, wherein the enzyme is a lipase, preferably an esterase or protease.

**3.** The process as claimed in claim 1, wherein

$R^1$ is unbranched or branched $C_1$-$C_8$-alkoxy, which is unsubstituted or mono- or polysubstituted by fluorine or chlorine or mono- or polysubstituted by $C_1$-$C_4$-alkoxy or by phenyl or phenyl which carries 1 to 3 substituents from the group comprising $C_1$-$C_4$-alkyl, $C_1$-$C_4$-alkoxy, halogen and nitro,

and

$R^2$ and $R^3$ are hydrogen, formyl, unbranched or branched ($C_1$-$C_{10}$-alkyl)carbonyl, which is unsubstituted or substituted in the alkyl moiety by one to three hydroxyl, halogen or phenyl radicals, which can be substituted by up to 3 radicals from the group comprising $C_1$-$C_4$-alkyl, $C_1$-$C_4$-alkoxy, halogen, nitro and $CF_3$, $C_1$-$C_4$-alkoxy and $C_1$-$C_4$-alkylthio, are benzoyl or benzoyl which is substituted by 1 to 3 radicals from the group comprising $C_1$-$C_4$-alkyl, $C_1$-$C_4$-alkoxy, halogen and nitro, or are another protective group customary for amino groups and known to the person skilled in the art, in particular selected from unbranched or branched ($C_1$-$C_{20}$-alkoxy)carbonyl and $C_1$-$C_{20}$-alkylsulfonyl, which in each case are unsubstituted or substituted in the alkyl moiety by one or more radicals from the group comprising hydroxyl, halogen, $C_1$-$C_4$-alkoxy, $C_1$-$C_4$-alkylthio, phenyl or phenyl which carries 1 to 3 substituents from the group comprising $C_1$-$C_{12}$-alkyl, $C_1$-$C_{12}$-alkoxy, halogen, nitro and $CF_3$, and phenylsulfonyl which can be substituted by up to three $C_1$-$C_{12}$-alkyl, $C_1$-$C_{12}$-alkoxy, halogen, nitro and $CF_3$ radicals, and is very particularly selected from unbranched or branched ($C_1$-$C_{10}$-alkoxy)carbonyl and $C_1$-$C_{20}$-alkylsulfonyl which in each case are unsubstituted or substituted in the alkyl moiety by one or more radicals from the group comprising hydroxyl, halogen, methoxy, ethoxy, phenyl or a phenyl radical which carries 1 to 3 substituents from the group comprising $C_1$-$C_4$-alkyl, $C_1$-$C_4$-alkoxy, halogen and nitro,

where $R^2$ in each case is equal or unequal to $R^3$.

**4.** The process as claimed in one or more of the abovementioned claims, wherein

$R^1$ is unbranched or branched $C_1$-$C_6$-alkoxy, preferably methoxy, ethoxy, isopropoxy or benzyloxy

and

$R^2$ and $R^3$ are hydrogen, formyl, unbranched or branched ($C_1$-$C_6$-alkyl)carbonyl, preferably acetyl, which can be unsubstituted or substituted in the alkyl moiety by one to three halogen or phenyl radicals, are benzoyl or benzoyl which is substituted by 1 to 3 radicals from the group comprising $C_1$-$C_4$-alkyl, $C_1$-$C_4$-alkoxy, halogen and nitro or are unbranched or branched ($C_1$-$C_4$-alkoxy )carbonyl, preferably methoxycarbonyl, tert-butoxycarbonyl (BOC) or benzyloxycarbonyl (Z) which can also be substituted in the phenyl ring by up to three radicals from the group comprising $C_1$-$C_4$-alkyl, $C_1$-$C_4$-alkoxy and halogen,

where

$R^2$ in each case is equal or unequal to $R^3$.

**5.** The process as claimed in one or more of the abovementioned claims, wherein $C_1$-$C_6$-alkyl is in particular methyl, ethyl, 1-propyl or 2-propyl, n-, i-, tert- or 2-butyl, 3-methylbut-2-yl, n-, i-, tert-, 2- or 3-pentyl, n-hexyl or a stereoisomeric hexyl, and $C_1$-$C_6$-alkoxy is in particular ($C_1$-$C_6$-alkyl)oxy, where the alkyl radical in this case has the abovementioned meaning.

**6.** The process as claimed in one or more of claims 1-5, wherein the enzymatic cleavage is carried out at a temperature of 10 to 80°C, preferably of 20 to 50°C.

**7.** The process as claimed in one or more of claims 1 to 6, wherein the enzymes are employed in free or immobilized form.

**8.** The process as claimed in one or more of claims 1 to 7, wherein the D-vinylglycine derivative of the formula I and the L-form of the vinylglycine derivative of the formula I where $R^1$ = OH are obtained by means of the enzymatic hydrolysis.

**9.** The process as claimed in one or more of claims 1 to 8, wherein the D-vinylglycine precursor of the formula II and the L-form of the vinylglycine derivative of the formula II where $R^1$ = OH are obtained by means of the enzymatic hydrolysis.